# EUROPEAN PATENT SPECIFICATION

(11) **EP 4 436 488 B1**
(45) Date of publication and mention of the grant of the patent: **18.11.2026**
(21) Application number: 22713123.2
(22) Date of filing: 03.03.2022
(51) Int. Cl.: A61B 17/04

(54) **KNOTLESS SUTURE CONSTRUCT FOR HARD BODY ANCHORS AND ALL SUTURE ANCHORS**
KNOTENLOSES NAHTKONSTRUKT FÜR HARTE KÖRPERANKER UND ALLE NAHTANKER
CONSTRUCTION DE SUTURE SANS NOEUD POUR ANCRAGES DE CORPS DURS ET TOUS LES ANCRAGES DE SUTURE

(30) Priority: 04.03.2021 US 202163156585 P; 04.06.2021 US 202163197009 P
(43) Date of publication of application: 02.10.2024
(73) Proprietor: CONMED Corporation, Largo, FL 33773 (US)
(72) Inventor: BRESLICH, Grady, Saint Petersburg, FL 33713 (US); ALFONSO, Gregory, Seffner, FL 33584 (US)
(74) Representative: Strehl & Partner mbB
(86) International application number: PCT/US2022/018770
(87) International publication number: WO 2022/187536

(56) References cited:
- AU-A1- 2019 227 635
- US-A1- 2017 049 432
- US-A1- 2018 221 010

## Description

### BACKGROUND OF THE INVENTION

### 1. FIELD OF THE INVENTION

The present invention relates to orthopedic bond anchors and, more specifically, to a knotless suture construct that can be used to secure tissue to a hard or soft bone anchor.

### 2. DESCRIPTION OF THE RELATED ART

During orthopedic surgeries, tissue may need to be attached to a specific location on a bone. Tying the tissue down to the bone is not optimal as the process leaves a knot stack at the location, which can interfere with other anatomy over time. Conventional knotless anchor approaches can avoid the knot stack but nevertheless require the anchor inserter to remain in position while the tissue is secured, thereby making it impossible for the tissue to be secured directly over the anchor insertion point. Accordingly, there is a need in the art for an approach that can fix tissue to bone at the location of the bone anchor without leaving a knot stack.
US 2018/221010 A1 discloses a tissue to bone system with the features in the preamble of present claim 1. Another conventional system is described in US 2017/049432 A1. AU 2019 227 635 A1 is directed generally to suture anchors and, more particularly, to a knotless suture anchor construct for securing a tissue in a desired position relative to a bone hole.

### BRIEF SUMMARY OF THE INVENTION

The present invention as defined in claim 1 provides a suture construct that can be used to secure tissue to bone at the location of the bone anchor without the need for a knot stack. The suture construct has a fixation limb including a body with a first end and a second end. A loop is formed at the first end of the body and interconnected to a tail extending out of the other end of the body. A suture shuttle having a shuttle loop and a shuttle tail extends through the body of fixation limb so that the shuttle loop is positioned proximately to the loop and the shuttle tail exits the body from a splice formed in an intermediate position of the body. The suture construct may be coupled to a hard body anchor, such as by forming a larks knot or luggage tag knot with the loop of the suture construct and an eyelet of the hard body bone anchor. The fixation limb of the suture construct extends through a longitudinal bore of the anchor so that the shuttle loop and shuttle tail extend out of the longitudinal bore of the anchor. The splice of the suture construct is positioned so that it will be just outside of the longitudinal bore of the anchor.

In another embodiment of the present invention, the suture construct may be coupled to an all suture anchor having a suture body that is configured to be compacted from an extended configuration to a compressed configuration. In this embodiment, the suture construct and the suture shuttle may be woven through the suture body of the all suture anchor so that the loop of the suture construct is positioned at a first end of the suture body and the tail of the suture construct, the shuttle loop and the shuttle tail are positioned at a second end of the suture body. The splice is positioned between the first end and the second end of the suture body when the suture body is in the extended configuration.

### BRIEF DESCRIPTION OF THE SEVERAL VIEWS OF THE DRAWING(S)

The present invention will be more fully understood and appreciated by reading the following Detailed Description in conjunction with the accompanying drawings, in which:
FIG. 1 is a schematic of a suture construct according to the present invention.
FIG. 2 is a schematic of a suture construct according to the present invention loaded into a hard body anchor.
FIG. 3 is a series of schematics showing the operation of a suture construct according to the present invention.
FIG. 4 is a schematic of first step of a suture construct according to the present invention securing tissue to a bone having an anchor loaded with the suture construct.
FIG. 5 is a schematic of another step of suture construct according to the present invention securing tissue to a bone having an anchor loaded with the suture construct.
FIG. 6 is a schematic of a first approach for loading a suture construct according to the present invention into a hard body anchor.
FIG. 7 is another schematic of a first approach for loading a suture construct according to the present invention into a hard body anchor.
FIG. 8 is a further schematic of a first approach for loading a suture construct according to the present invention into a hard body anchor.
FIG. 9 is an additional schematic of a first approach for loading a suture construct according to the present invention into a hard body anchor.
FIG. 10 is schematic of a second approach for loading a suture construct according to the present invention into a hard body anchor.
FIG. 11 is a further schematic of a second approach for loading a suture construct according to the present invention into a hard body anchor.
FIG. 12 is an additional schematic of a second approach for loading a suture construct according to the present invention into a hard body anchor.
FIG. 13 is a schematic of another approach for loading a suture construct according to the present invention.
FIG. 14 a schematic of the approach of FIG. 13 after the fixation limb has been shuttled through the anchor.
FIG. 15 is a schematic of an approach for loading a suture construct according to the present invention into an all suture anchor.
FIG. 16 is another schematic of an approach for loading a suture construct according to the present invention into an all suture anchor.
FIG. 17 is a schematic of an approach for securing tissue to bone using a suture construct according to the present invention with an all suture anchor.
FIG. 18 is another schematic of an approach for securing tissue to bone using a suture construct according to the present invention with an all suture anchor.
FIG. 19 is a schematic of another approach for securing tissue to bone using a suture construct according to the present invention with an all suture anchor.
FIG. 20 is a schematic of another approach for securing tissue to bone using a suture construct according to the present invention with an all suture anchor.
FIG. 21 is a schematic of another approach for securing tissue to bone using a suture construct according to the present invention with an all suture anchor.
FIG. 22 is a schematic of another approach for securing tissue to bone using a suture construct according to the present invention with an all suture anchor.
FIG. 23 is a schematic of another approach for securing tissue to bone using a suture construct according to the present invention with an all suture anchor.
FIG. 24 is a schematic of another approach for securing tissue to bone using a suture construct according to the present invention with an all suture anchor.
FIG. 25 is a schematic of another approach for securing tissue to bone using a suture construct according to the present invention with an all suture anchor.
FIG. 26 is a schematic of another approach for securing tissue to bone using a suture construct according to the present invention with an all suture anchor
FIG. 27 is a schematic of securing tissue to bone using a second embodiment of a suture construct according to the present invention.
FIG. 28 is another schematic of securing tissue to bone using a second embodiment of a suture construct according to the present invention

### DETAILED DESCRIPTION OF THE INVENTION

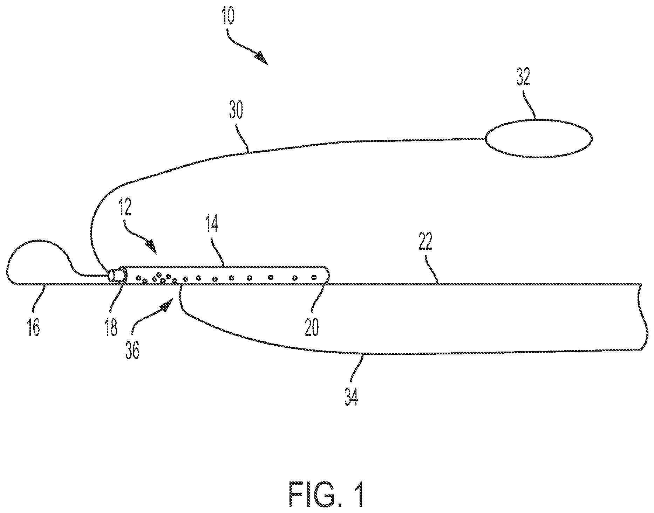

Referring to the drawings, wherein like numerals refer to like parts throughout, there is seen in FIG. 1 a suture construct 10 according to the present invention. Suture construct 10 comprises a fixation limb 12 having a body 14 with a loop 16 formed at a first end 18 of body 14 that extends back through body 14 as a bury to emerge at a second end 20 as a tail 22. Fixation limb 12 may be formed from a single length of suture that is spliced at one end to define first end 18 of body 14 and then burying the spliced end back through body 14 to emerge as tail 22 at second end 20 and thus form loop 16 at first end 18 having a predetermined diameter with a bury extending through body 14. Tail 22 may be pulled further through body 14 to reduce the diameter of loop 16. Tail 22 is used to entrap tissue to be fixed to a bone, as will be explained herein.

Suture construct 10 further comprises a suture shuttle 30 having a shuttle loop 32 and a shuttle tail 34 that extends through the center of fixation limb 12 and exits from a splice 36 in body 14 so that shuttle loop 32 is positioned at first end 18 proximate to loop 16. Positioning of splice 36 is configured to position the emergence of fixation limb 12 proximate to the surface of the bone to which suture construct 10 is being used, and is thus dependent on the nature and dimensions of a hard body anchor or all suture anchor used with suture construct 10, as described in further detail below.

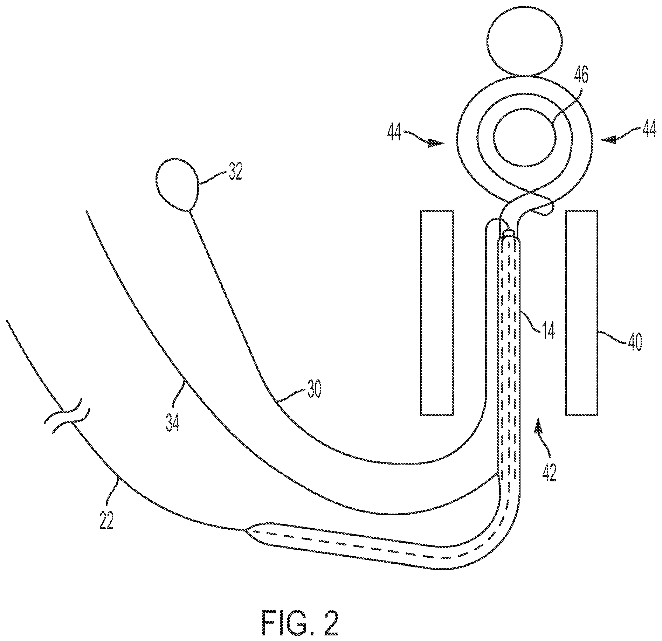

Referring to FIG. 2, suture construct 10 may be used in connection with a hard body anchor 40 having a longitudinal bore 42 and lateral openings 44 that combined with a post 46 therebetween to form an eyelet. In this embodiment, suture construct 10 may be secured to post 46 of anchor 40 by forming a lark head knot (also known as a luggage tag knot) where loop 16 is passed around post 46 and the rest of suture construct 10 passes through loop 16 extends longitudinally through bore 42 of hard body anchor 40 so that fixation limb 12 as well as shuttle loop 32 and shuttle tail 34 extend from hard body anchor 40. Shuttle loop 32 is also outside of hard body anchor 40 but extends into body 14 proximately to post 46. Suture shuttle 30 is positioned in hard body anchor 40 so that shuttle tail 34 exits splice 36 outside of bore 42 and thus outside of hard body anchor 40. Thus, the location of splice 36 in fixation limb 12 is determined according to the dimensions of post 46 and longitudinal bore 42 of hard body anchor 40. For example, in a hard body anchor 40 of 2.5 millimeter or 3.0 millimeter outer diameter and a length of 9.8 millimeters or 10.6 millimeter, body 14 may have a length of approximately 178 millimeters with splice 36 located seven millimeters (+/- 1 mm) from first end 18.

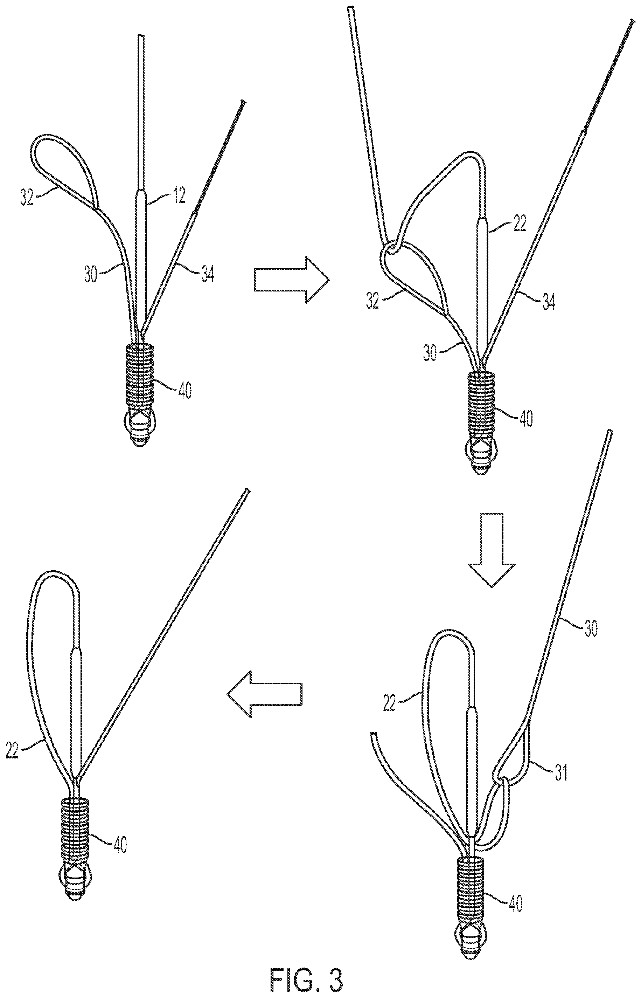

Referring to FIG. 3, once fixation limb 12 as well as shuttle loop 32 and shuttle tail 34 have been positioned to extend from hard body anchor 40, fixation limb 12 may be passed through shuttle loop 32. Shuttle tail 34 is then pulled away from hard body anchor 40 until shuttle loop 32 is drawn through splice 36, through body 14, and then out of suture construct 10. Movement of shuttle loop 32 through splice 36 and body 14 draws fixation limb 12 through splice 36 and body 14 so that fixation limb 12 is pulled back through splice 36 and body 14 until tail 22 is released, thereby forming a one-way tightening loop that can be drawn closed by pulling tail 22.

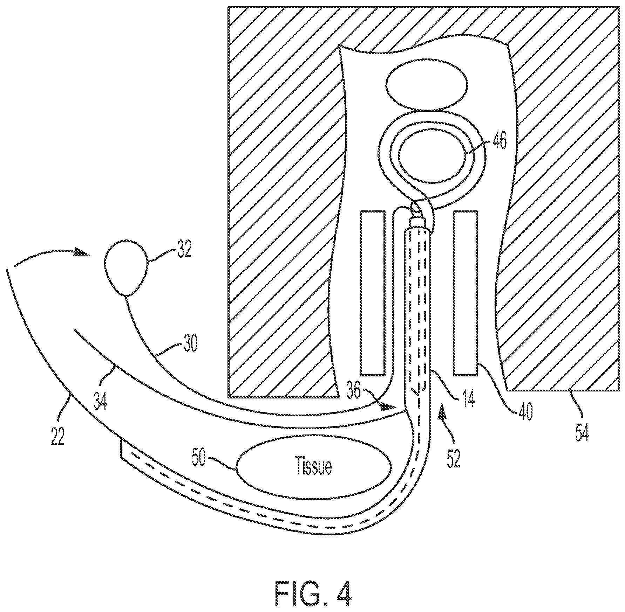

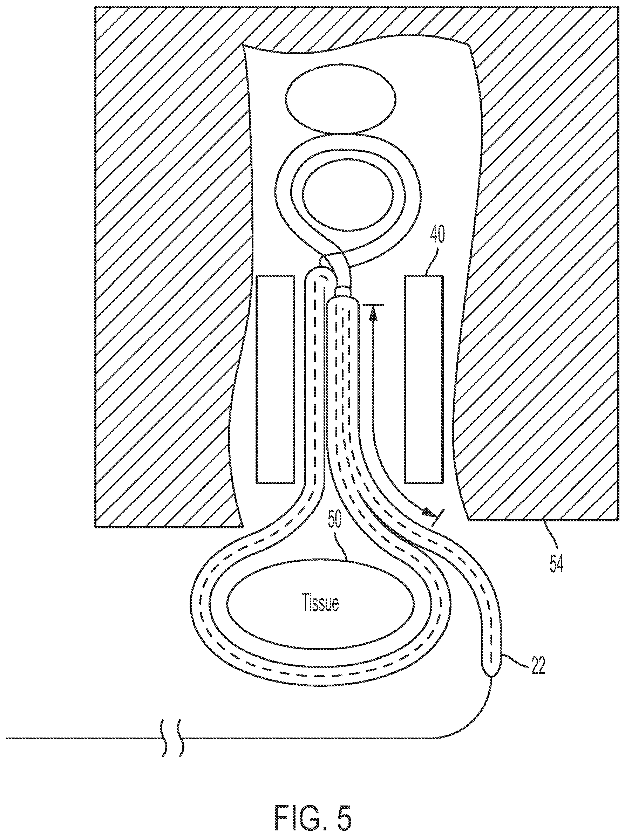

Referring to FIGS. 4 and 5, the operation of suture construct 10 depicted in FIG. 3 may be performed after hard body anchor 40 has been installed into a drilled hole 52 in a bone 54 in the conventional approach for seating hard body anchor 40. As discussed above, splice 36 is located along fixation limb 12 so that it will be positioned just outside of hard body anchor 40, such as between one and two millimeters, and thus slightly above hole 52 in bone 54. Fixation limb 12 may then be passed over tissue 50 to be secured to bone 54 and loaded through shuttle loop 32. Shuttle tail 34 may then be pulled to withdraw shuttle loop 32 and the captured fixation limb 12 into longitudinal bore 42 and shuttle the fixation limb 12 through body 14 and out of splice 36. Fixation limb 12 has thus formed one-way tightening loop around tissue 50 as any force applied to suture construct by tissue that would otherwise tend to loosen fixation limb 14 will further tighten body 14 about fixation limb 12. Fixation limb 12 can be further pulled to tighten loop and then trimmed to leave tissue 50 secured to bone 54 adjacent to hard body anchor 40 without the use of any knots.

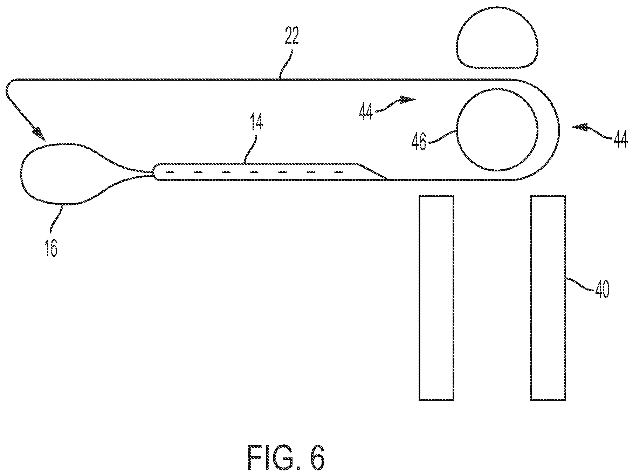

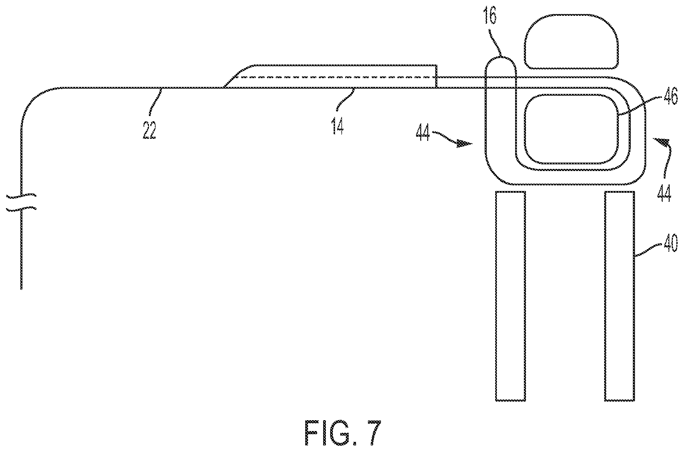

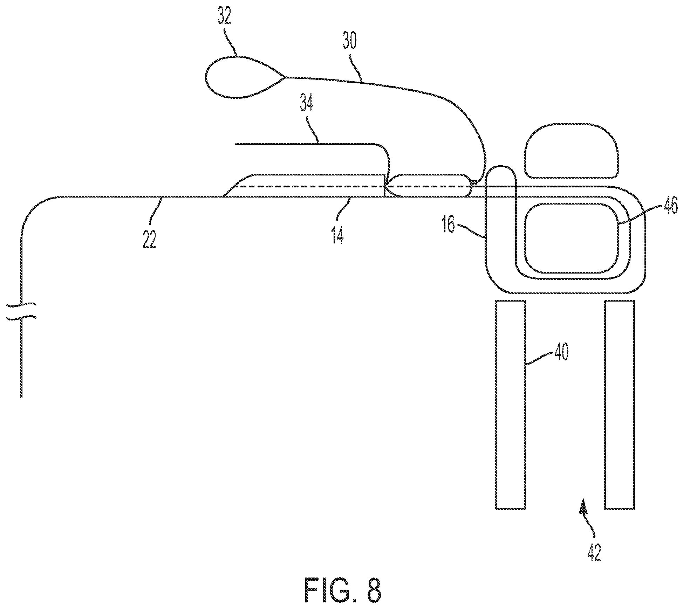

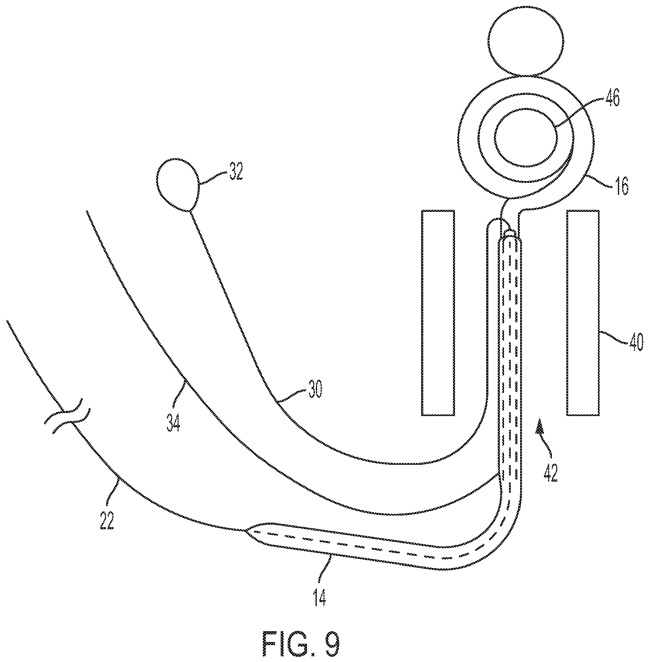

Referring to FIG. 6, suture construct 10 may be coupled to hard body anchor 40 by passing fixation limb 12 around post 46 of eyelet. As seen in FIG. 7, tail 22 of fixation limb 12 may be passed through loop 16 to form a larks knot (or luggage tag knot) about post 46. Tail 22 is then pulled to close loop 16 tightly around post 46 of eyelet. As seen in FIG. 8, suture shuttle 30 may then be spliced to body 14 as described above so that splice 36 will be positioned just proximate to anchor 40 when fully installed. As seen in FIG. 9, fixation limb 12 and suture shuttle 30 (including shuttle loop 32 and shuttle tail 34) may then be passed through bore 42 of hard body anchor 40 so that tail 22 along with suture shuttle 30 extend from hard body anchor 40 and splice 36 is just outside of bore 42.

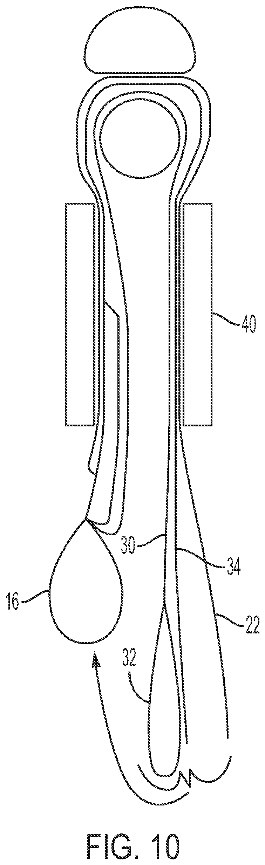

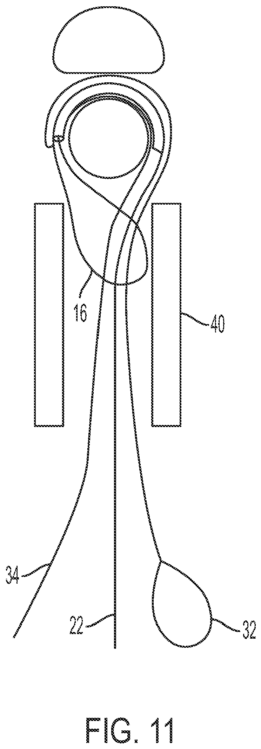

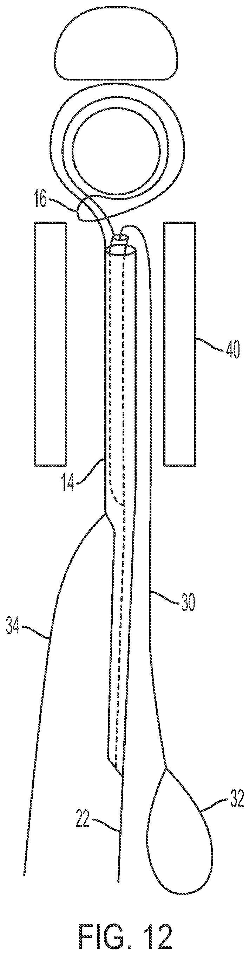

Referring to FIG. 10, suture construct 10 may also be coupled to hard body anchor 40 by forming suture construct 10 and then passing suture construct 10 through longitudinal bore 42 of hard body anchor 40, around post 46 of eyelet and back through longitudinal bore 42 so that loop 16 as well as tail 22, shuttle loop 32 and shuttle tail 34 extend out of hard body anchor 40. Tail 22, shuttle loop 32 and shuttle tail 34 may then be passed through loop 16 and tensioned, as seen in FIG. 11, to form a larks knot (or luggage tag knot) about post 46, as seen in FIG. 12.

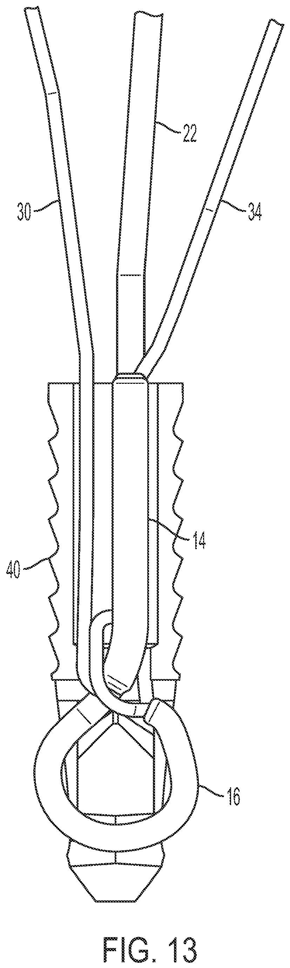

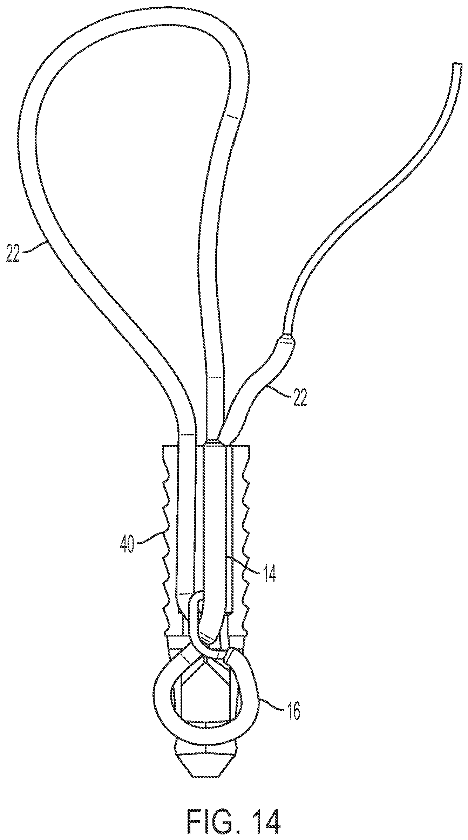

Referring to FIG. 13 suture construct 10 may be coupled to hard body anchor 10 so that shuttle loop 32 and shuttle tail 34 extend through loop 16 forming the larks knot (or luggage tag knot). As seen in FIG. 14, shutting of fixation limb 12 will result in fixation limb being drawn through loop 16 of the larks knot (or luggage tag knot) when shuttled back through anchor 10, which will reduce loop 16 to the point that loop 16 will hold, as shown by the thick-to-thin transition on the right side of FIG. 14 though the splice, resulting in the maximum amount of material in the splice.

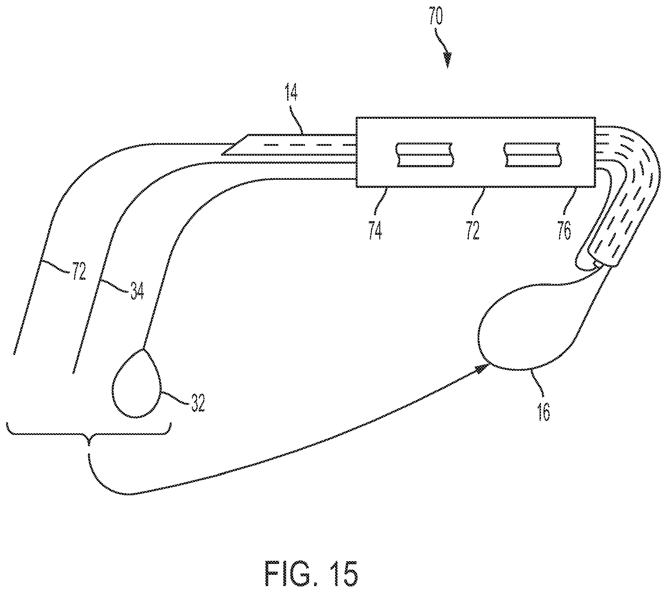

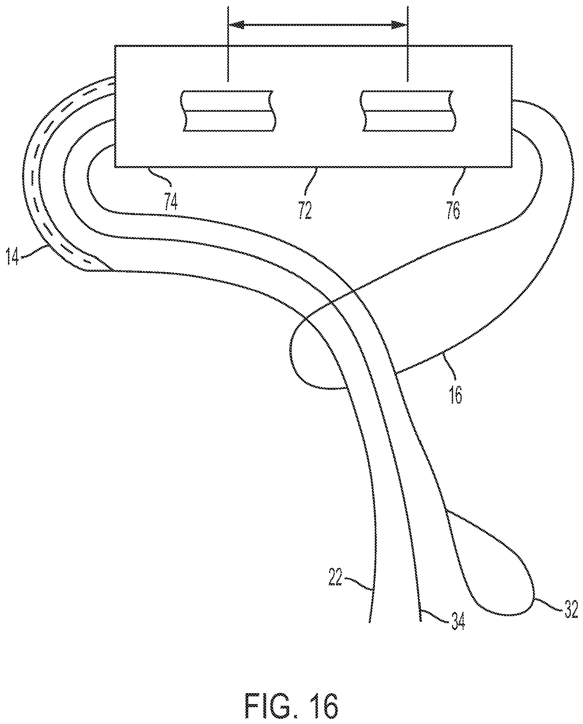

Referring to FIG. 15, suture construct 10 may also be used in connection with an all suture anchor 70 comprising a suture body 72 formed from a braided construct that extends from a first end 74 to a second end 76 and that can be compressed from an extended position to a compressed position, where suture body 72 forms a spheroid that is secured in hole 52 of bone 54. In this embodiment, suture construct 10 is woven through suture body 72 of all suture anchor 70 so that tail 22, shuttle loop 32 and shuttle tail 34 extend from first end 74 while loop 16 extends from second end 76 of suture anchor 70. Referring to FIG. 16, tail 22, shuttle loop 32 and shuttle tail 34 are passed through loop 16 prior to insertion of all suture anchor 70 in hole 52 of bone 54, and splice 36 is positioned between first end 74 and second end 76 of suture body 72. Loop 16 thus form a larks knot or luggage tag knot with suture body 72, but is left loose at this point so that suture body 72 remains in its extended position.

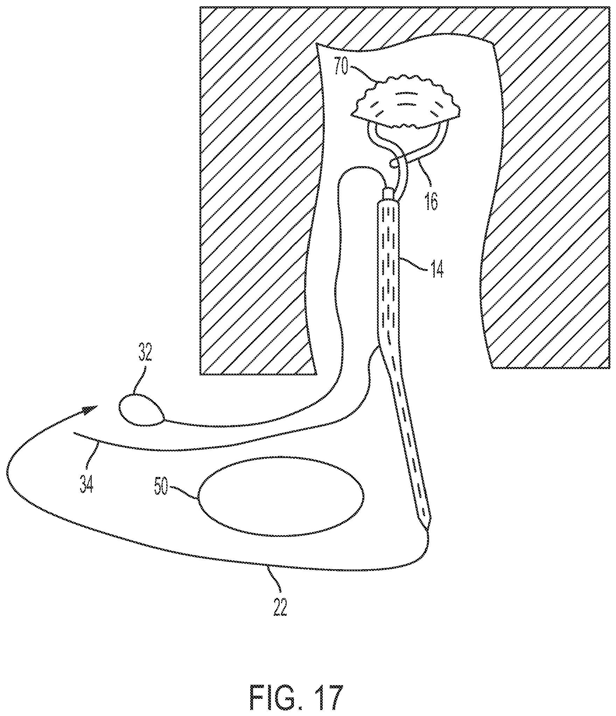

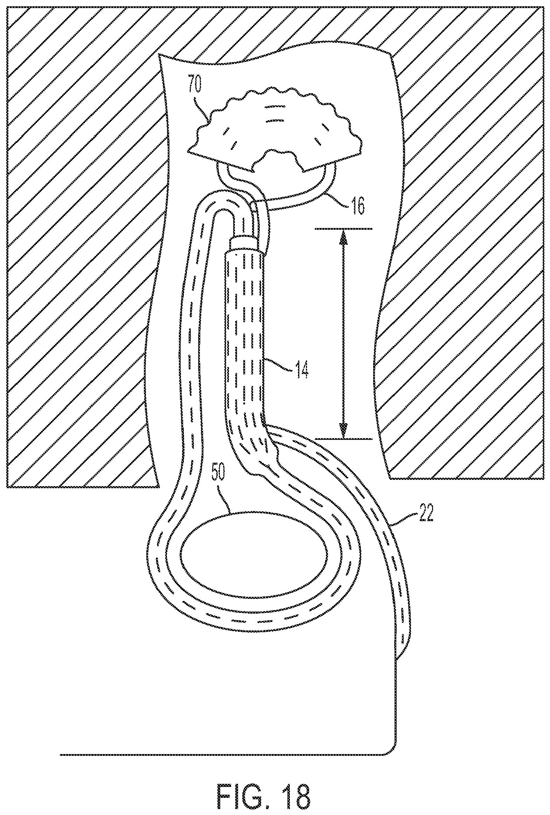

Referring to FIG. 17, all suture anchor 70 containing suture construct 10 may be inserted into hole 52 of bone 54 so that suture body 72 including splice 36 and loop 16 are positioned in hole 52, and tail 22, shuttle loop 32 and shuttle tail 34 extend out of hole 52 in bone 54. Tail 22, shuttle loop 32 and shuttle tail 34 are pulled to deploy all suture anchor 70, which results in suture body 72 being compressed into its spheroid configuration. Body 14 of fixation limb 12 is now fully positioned outside of all suture anchor 70 with just loop 16 forming a larks knot or luggage tag knot with suture body 72. Tail 22 may then be extended over tissue 50 to be anchored and passed through shuttle loop 32. Shuttle tail 34 may then be pulled to draw fixation limb 12 through body 14 and out of splice 36, as seen in FIG. 18, thereby forming one-way tightening loop around tissue 50 is the same manner as described above with respect to hard body anchor 40.

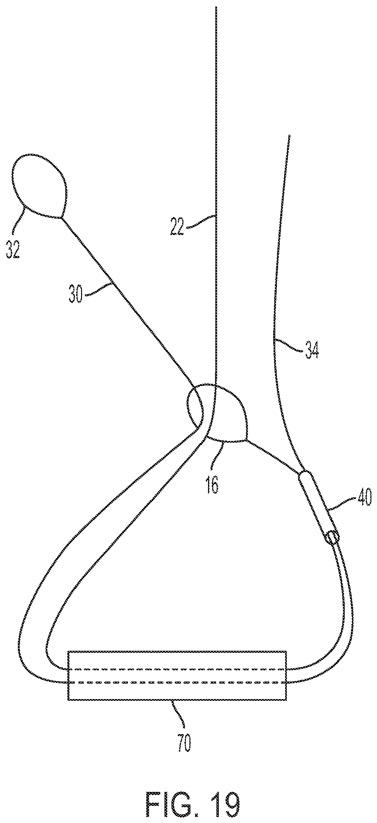

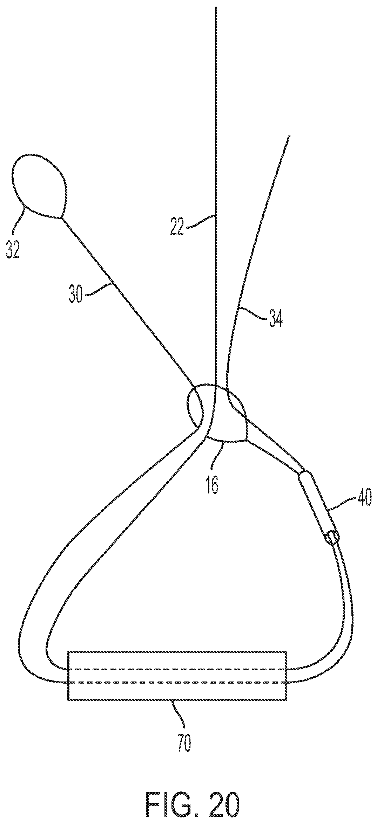

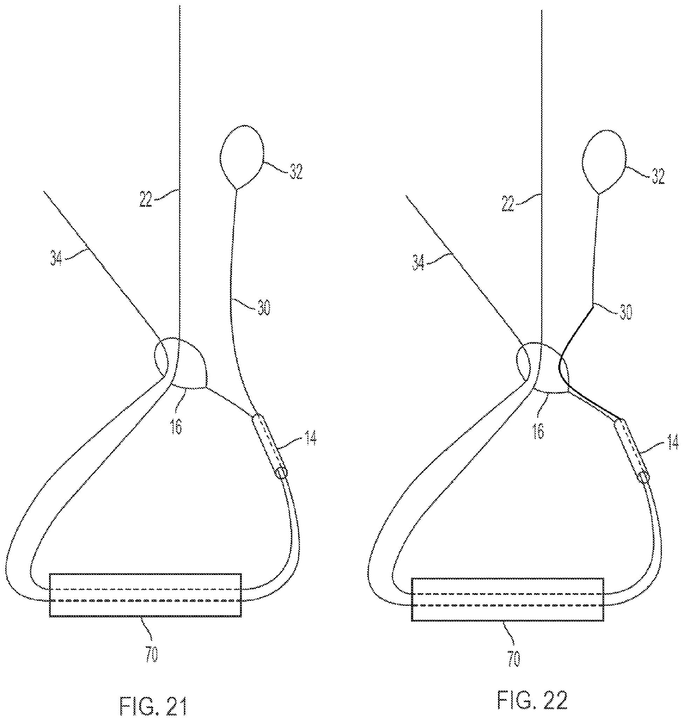

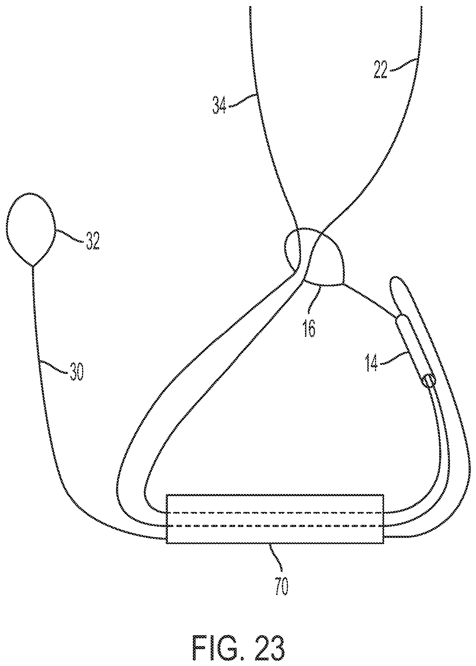

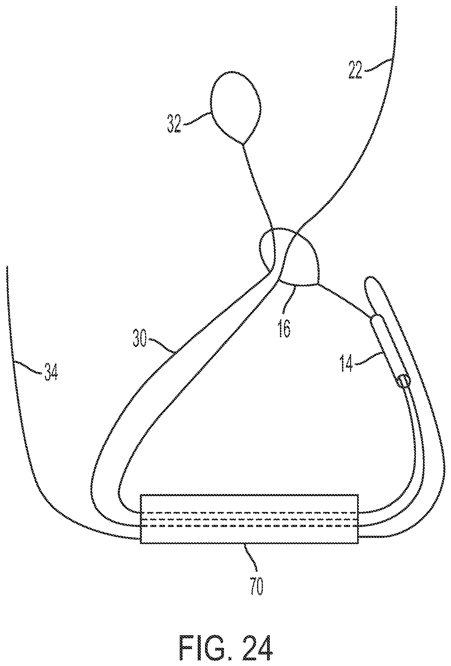

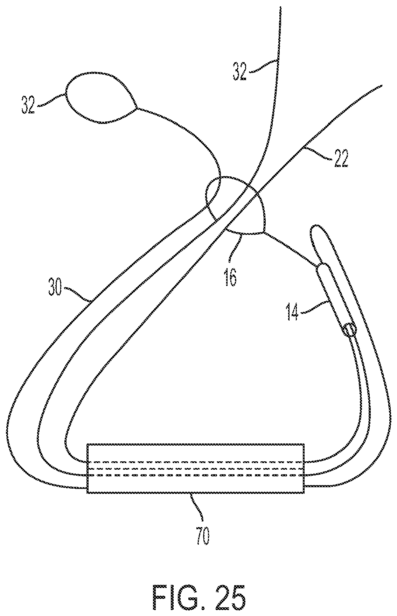

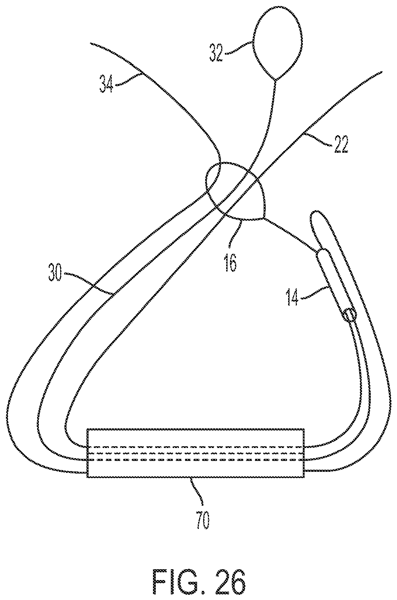

FIG. 19 through 26 illustrate the various ways in which loop 16, fixation limb 12, tail 22, shuttle loop 32, and shuttle tail 34 may be arranged relative to suture body 72 for deployment in hole 52 of bone 54 as described above.

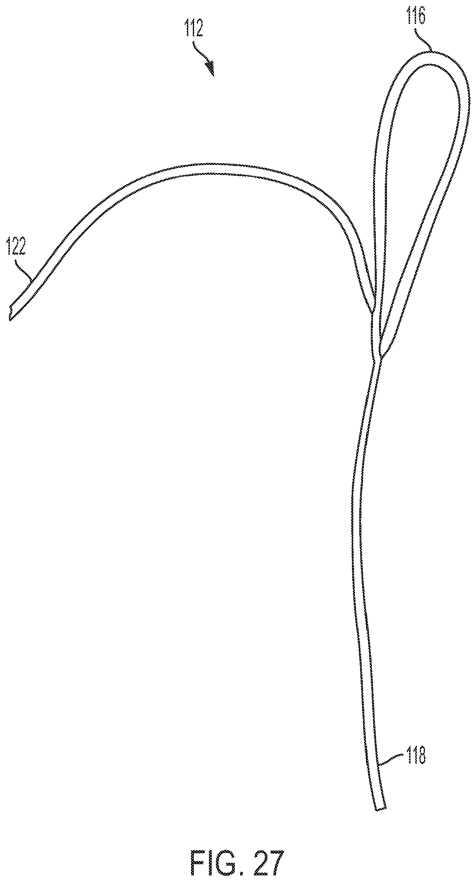

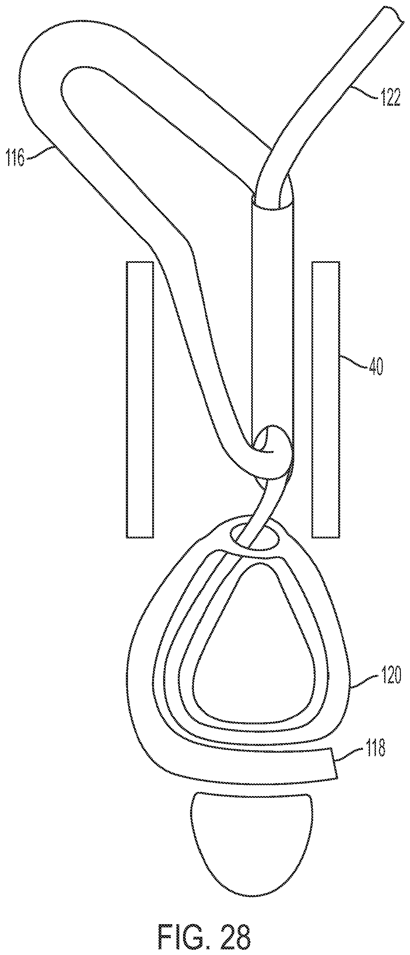

Referring to FIG. 27, another embodiment of a suture construct according to the present invention comprises a fixation limb 112 having a body that is folded and spliced back through a portion of body to form a tissue fixation loop 116 and a loop-reducing end 122 proximate to loop 116 as well as a fixed end 118 at the other end of body. Tensioning of loop-reducing end 122 will thus reduce the diameter of loop 116, while splicing back through body resists opening of loop 116 so that tissue fixing loop 116 can only be closed. Suture construct may be secured to hard body anchor 40 by securing fixed end 118 back to body or by passing fixed end 118 through itself to form an anchor loop 120 that may then be used to secure suture construct to a hard body anchor 40, such as by forming a larks knot or luggage tag knot using anchor loop 120. Tissue fixing loop 116 can thus be used to capture and secure tissue directly over hard body anchor 40 after the anchor driver has been removed from the site, as seen in FIG. 28.

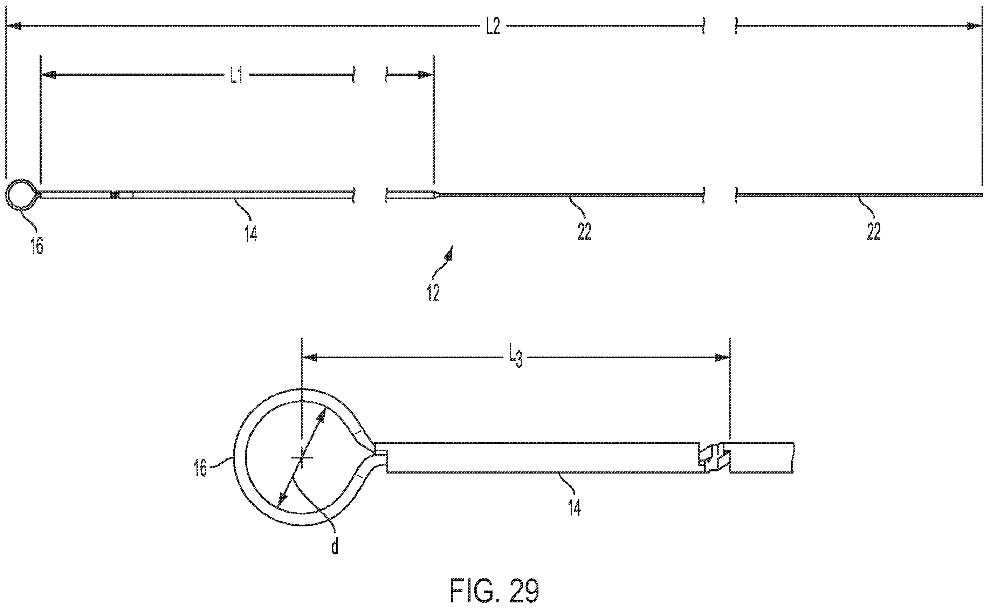

Referring to FIG. 29, fixation limb 12 may be formed from a single suture that is braided as a unitary structure where loop 16 is formed as a round braided section of seventeen fibers, body 14 is formed from a flat braided section of seventeen fibers to be flat, and tail 22 is formed from a round braided section with a gradually reduced number of fibers down to eleven or less. Loop 16 may have a diameter (d) of about 2.33 mm, body 14 may have a length (L1) of about 152 mm +/- 12.7, and tail 22 may have a length (L2) so that the overall length of fixation limb 12 is has a length (L3) of about 660 mm +/- 25.4 mm.

## Claims

1. A system for securing an amount of tissue (50) to a bone (54), comprising:
a suture construct (10) formed by a fixation limb (12) having a body (14) with a first end (18) and a second end (20); and
a suture shuttle (30) having a shuttle loop (32) and a shuttle tail (34) extending into and through the body (14) of the fixation limb (12) so that the shuttle tail (34) exits the body (14) from an exit splice location,
wherein
the suture construct (10) has a loop (16) formed at the first end (18) of the body (14), and a tail (22) extending from the loop (16) through the body (14), and the shuttle loop (32) is positioned proximately to the loop (16) of the suture construct (10), **characterised in that**
the fixation limb (12) is formed from a single length of suture that is spliced at one end to define the first end (18) of the body (14) and then burying the spliced end back through the body (14) to emerge as the tail (22) at the second end (20) and thus form the loop (16) at the first end (18) having a predetermined diameter with a bury extending through the body (14), so that the tail (22) is configured to be pulled further through the body (14) to reduce the diameter of the loop (16).

2. The system of claim 1, further comprising a hard body anchor (40) coupled to the suture construct (10).

3. The system of claim 2, where the hard body anchor (40) is coupled to the suture construct (10) by the loop (16) and an eyelet of the hard body anchor (40).

4. The system of claim 3, wherein the loop (16) is formed into a luggage tag knot with the eyelet.

5. The system of claim 4, wherein the fixation limb (12) of the suture construct (10) extends through a longitudinal bore (42) of the hard body anchor (40).

6. The system of claim 5, wherein the shuttle loop (32) and shuttle tail (34) extend through the longitudinal bore (42) of the hard body anchor (40).

7. The system of claim 6, wherein the exit splice location is positioned outside of the longitudinal bore (42) of the hard body anchor (40).

8. The system of claim 1, further comprising an all suture anchor (70) coupled to the suture construct (10).

9. The system of claim 8, wherein the all suture anchor (70) includes a suture body (72) that is configured to be compacted from an extended configuration to a compressed configuration.

10. The system of claim 9, wherein the suture construct (10) and the suture shuttle (30) is woven through the suture body (72) of the all suture anchor (70) so that the loop (16) of the suture construct (10) is positioned at a first end (74) of the suture body (72) and one or more of the tail (22) of the suture construct (10), the shuttle loop (32), and the shuttle tail (34) are positioned at a second end (76) of the suture body (72).

11. The system of claim 10, wherein one of more of the tail (22) of the suture construct (10), the shuttle loop (32), and the shuttle tail (34) extend through the loop (16) of the suture construct (10).

12. The system of claim 11, wherein the tail (22) of the suture construct (10) and the shuttle tail (34) extend through the loop (16) of the suture construct (10).

13. The system of claim 12, wherein all of the tail (22) of the suture construct (10), the shuttle loop (32), and the shuttle tail (34) extend through the loop (16) of the suture construct (10).

## Patentansprüche

1. System zum Befestigen einer Gewebemenge (50) an einem Knochen (54), umfassend:
ein Nahtkonstrukt (10), das durch einen Fixierungsschenkel (12) mit einem ein erstes Ende (18) und ein zweites Ende (20) aufweisenden Körper (14) gebildet ist; und
einen Nahtshuttle (30) mit einer Shuttle-Schlaufe (32) und einem Shuttle-Schwanz (34), die sich in den Körper (14) des Fixierungsschenkels (12) und durch diesen erstrecken, so dass der Shuttle-Schwanz (34) den Körper (14) an einer Austritts-Spleißstelle verlässt,
wobei das Nahtkonstrukt (10) eine Schlaufe (16) aufweist, die an dem ersten Ende (18) des Körpers (14) gebildet ist, und einen Schwanz (22), der sich von der Schlaufe (16) durch den Körper (14) erstreckt, und die Shuttle-Schlaufe (32) nahe der Schlaufe (16) des Nahtkonstrukts (10) positioniert ist,
**dadurch gekennzeichnet, dass** der Fixierungsschenkel (12) aus einer einzelnen Nahtlänge gebildet ist, die an einem Ende gespleißt ist, um das erste Ende (18) des Körpers (14) zu definieren, und dann das gespleißte Ende zurück durch den Körper (14) vergraben wird, um als der Schwanz (22) an dem zweiten Ende (20) hervorzutreten und somit die Schlaufe (16) an dem ersten Ende (18) mit einem vorbestimmten Durchmesser mit einem sich durch den Körper (14) erstreckenden Vergrabungsabschnitt zu bilden, so dass der Schwanz (22) dazu konfiguriert ist, weiter durch den Körper (14) gezogen zu werden, um den Durchmesser der Schlaufe (16) zu verringern.

2. System nach Anspruch 1, ferner umfassend einen harten Körperanker (40), der mit dem Nahtkonstrukt (10) gekoppelt ist.

3. System nach Anspruch 2, wobei der harte Körperanker (40) mit dem Nahtkonstrukt (10) durch die Schlaufe (16) und eine Öse des harten Körperankers (40) gekoppelt ist.

4. System nach Anspruch 3, wobei die Schlaufe (16) mit der Öse zu einem Kofferanhänger-Knoten gebildet ist.

5. System nach Anspruch 4, wobei sich der Fixierungsschenkel (12) des Nahtkonstrukts (10) durch eine Längsbohrung (42) des harten Körperankers (40) erstreckt.

6. System nach Anspruch 5, wobei sich die Shuttle-Schlaufe (32) und der Shuttle-Schwanz (34) durch die Längsbohrung (42) des harten Körperankers (40) erstrecken.

7. System nach Anspruch 6, wobei die Austritts-Spleißstelle außerhalb der Längsbohrung (42) des harten Körperankers (40) positioniert ist.

8. System nach Anspruch 1, ferner umfassend einen Nahtanker (70), der mit dem Nahtkonstrukt (10) gekoppelt ist.

9. System nach Anspruch 8, wobei der Nahtanker (70) einen Nahtkörper (72) umfasst, der dazu konfiguriert ist, von einer ausgefahrenen Konfiguration zu einer komprimierten Konfiguration kompaktiert zu werden.

10. System nach Anspruch 9, wobei das Nahtkonstrukt (10) und der Nahtshuttle (30) durch den Nahtkörper (72) des Nahtankers (70) gewoben sind, so dass die Schlaufe (16) des Nahtkonstrukts (10) an einem ersten Ende (74) des Nahtkörpers (72) positioniert ist und einer oder mehrere von dem Schwanz (22) des Nahtkonstrukts (10), der Shuttle-Schlaufe (32) und dem Shuttle-Schwanz (34) an einem zweiten Ende (76) des Nahtkörpers (72) positioniert sind.

11. System nach Anspruch 10, wobei sich einer oder mehrere von dem Schwanz (22) des Nahtkonstrukts (10), der Shuttle-Schlaufe (32) und dem Shuttle-Schwanz (34) durch die Schlaufe (16) des Nahtkonstrukts (10) erstrecken.

12. System nach Anspruch 11, wobei sich der Schwanz (22) des Nahtkonstrukts (10) und der Shuttle-Schwanz (34) durch die Schlaufe (16) des Nahtkonstrukts (10) erstrecken.

13. System nach Anspruch 12, wobei sich alle von dem Schwanz (22) des Nahtkonstrukts (10), der Shuttle-Schlaufe (32) und dem Shuttle-Schwanz (34) durch die Schlaufe (16) des Nahtkonstrukts (10) erstrecken.

## Revendications

1. Système pour immobiliser une quantité de tissu (50) sur un os (54), comprenant :
une construction (10) de suture formée par un élément (12) de fixation ayant un corps (14) avec une première extrémité (18) et une deuxième extrémité (20) ; et
une navette (30) de suture ayant une boucle (32) de navette et une queue (34) de navette s'étendant dans et à travers le corps (14) de l'élément (12) de fixation de telle sorte que la queue (34) de navette sort du corps (14) depuis un emplacement d'épissure de sortie,
dans lequel la construction (10) de suture a une boucle (16) formée à la première extrémité (18) du corps (14), et une queue (22) s'étendant depuis la boucle (16) à travers le corps (14), et la boucle (32) de navette est positionnée à proximité de la boucle (16) de la construction (10) de suture,
**caractérisé en ce que**
l'élément (12) de fixation est formé à partir d'une unique longueur de suture qui est épissée à une extrémité pour définir la première extrémité (18) du corps (14) et ensuite en réintégrant l'extrémité épissée à travers le corps (14) pour qu'elle émerge comme la queue (22) à la deuxième extrémité (20) et forme ainsi la boucle (16) à la première extrémité (18) ayant un diamètre prédéterminé avec un creux s'étendant à travers le corps (14), de telle sorte que la queue (22) est configurée pour être tirée plus avant à travers le corps (14) pour réduire le diamètre de la boucle (16).

2. Système selon la revendication 1, comprenant en outre un ancrage (40) de corps dur couplé à la construction (10) de suture.

3. Système selon la revendication 2, dans lequel l'ancrage (40) de corps dur est couplé à la construction (10) de suture par la boucle (16) et un œillet de l'ancrage (40) de corps dur.

4. Système selon la revendication 3, dans lequel la boucle (16) est formée en un nœud d'étiquette de bagage avec l'œillet.

5. Système selon la revendication 4, dans lequel l'élément (12) de fixation de la construction (10) de suture s'étend à travers un alésage longitudinal (42) de l'ancrage (40) de corps dur.

6. Système selon la revendication 5, dans lequel la boucle (32) de navette et la queue (34) de navette s'étendent à travers l'alésage longitudinal (42) de l'ancrage (40) de corps dur.

7. Système selon la revendication 6, dans lequel l'emplacement d'épissure de sortie est positionné à l'extérieur de l'alésage longitudinal (42) de l'ancrage (40) de corps dur.

8. Système selon la revendication 1, comprenant en outre un ancrage (70) toutes sutures couplé à la construction (10) de suture.

9. Système selon la revendication 8, dans lequel l'ancrage (70) toutes sutures inclut un corps (72) de suture qui est configuré pour être compacté d'une configuration étendue à une configuration compressée.

10. Système selon la revendication 9, dans lequel la construction (10) de suture et la navette (30) de suture sont tissées à travers le corps (72) de suture de l'ancrage (70) toutes sutures de telle sorte que la boucle (16) de la construction (10) de suture est positionnée à une première extrémité (74) du corps (72) de suture et une ou plusieurs parmi la queue (22) de la construction (10) de suture, la boucle (32) de navette, et la queue (34) de navette sont positionnées à une deuxième extrémité (76) du corps (72) de suture.

11. Système selon la revendication 10, dans lequel une ou plusieurs parmi la queue (22) de la construction (10) de suture, la boucle (32) de navette, et la queue (34) de navette s'étendent à travers la boucle (16) de la construction (10) de suture.

12. Système selon la revendication 11, dans lequel la queue (22) de la construction (10) de suture et la queue (34) de navette s'étendent à travers la boucle (16) de la construction (10) de suture.

13. Système selon la revendication 12, dans lequel la totalité de la queue (22) de la construction (10) de suture, de la boucle (32) de navette, et de la queue (34) de navette s'étendent à travers la boucle (16) de la construction (10) de suture.
